Europäisches Patentamt

European Patent Office

Office européen des brevets

(19)

(11) Veröffentlichungsnummer : **0 181 491**
**B1**

(12) **EUROPÄISCHE PATENTSCHRIFT**

(45) Veröffentlichungstag der Patentschrift :
28.06.89

(21) Anmeldenummer : 85112725.8

(22) Anmeldetag : 08.10.85

(51) Int. Cl.⁴ : **B 01 J 19/24//** C07H21/00,
C07K1/00

(54) Vorrichtung zur parallelen Durchführung einer Vielzahl von chemischen Reaktionssequenzen.

(30) Priorität : 18.10.84 CH 4994/84

(43) Veröffentlichungstag der Anmeldung :
21.05.86 Patentblatt 86/21

(45) Bekanntmachung des Hinweises auf die Patenterteilung : 28.06.89 Patentblatt 89/26

(84) Benannte Vertragsstaaten :
AT BE CH DE FR GB IT LI NL SE

(56) Entgegenhaltungen :
EP—A— 0 164 206
US—A— 3 876 881
US—A— 4 483 964
Die Akte enthält technische Angaben, die nach dem
Eingang der Anmeldung eingereicht wurden und die
nicht in dieser Patentschrift enthalten sind.

(73) Patentinhaber : F. HOFFMANN-LA ROCHE & CO.
Aktiengesellschaft
CH-4002 Basel (CH)

(72) Erfinder : Bannwarth, Wilhelm, Dr.
Liebenbergstrasse 8
D-7888 Rheinfelden-Beuggen. (DE)
Erfinder : Iaiza, Patrick
22 Rue des Maraichers
F-68300 St.Louis (FR)

(74) Vertreter : Körber, Wolfhart, Dr. et al
Patentanwälte Dipl.-Ing. H. Mitscherlich Dipl.-Ing. K.
Gunschmann Dr.rer.nat. W. Körber Dipl.Ing. J.
Schmidt-Evers Dipl.-Ing. W. Melzer Steinsdorfstrasse
10
D-8000 München 22 (DE)

## Beschreibung

Die Erfindung betrifft eine Vorrichtung zur Durchführung einer Vielzahl von Reaktionssequenzen. Eine derartige Vorrichtung eignet sich speziell zur Synthese von Polymermoleküle wie Nukleotiden, Proteinen etc. an einem Trägermaterial wie z. B. Glas, Kieselgel oder anderem geeigneten Material.

In der Regel geschieht der chemische Aufbau solcher Polymermoleküle in Reaktionskammern (z. B. Fritten, Säulen, etc.) in welchen der erste Baustein der zu synthetisierenden Moleküle an das entsprechende Polymermaterial gebunden vorliegt und die Zudosierung der zur Synthese erforderlichen Reagenzien entweder manuell oder automatisch erfolgen kann.

Es liegt in der Natur der Reaktionssequenzen, dass zum Aufbau eines längerkettigen Moleküls verhältnismässig viel Zeit benötigt wird. Benötigt man eine Vielzahl verschiedener, aus den gleichen Bausteinen aufgebaute Polymermoleküle, so ist der Zeitaufwand, bis alle gewünschten Syntheseprodukte vorliegen, oft unerträglich lang. Daraus ergibt sich ein Bedürfnis nach einer Möglichkeit, eine Vielzahl von verschiedenen Polymermolekülen aus gleichen Reagenzien simultan aufzubauen. In diesem Sinne liegt der Erfindung die Aufgabe zugrunde, eine Vorrichtung bereitzustellen mit der eine Vielzahl von Reaktionssequenzen simultan durchgeführt werden kann.

Erfindungsgemäss wurde dies durch die im Patentanspruch definierte Vorrichtung erreicht.

Die Vorrichtung dient speziell zur simultanen Synthese einer Vielzahl von DNA- und RNA-Segmenten unterschiedlicher Kettenlänge und Sequenz an einem polymeren Trägermaterial. In an sich bekannter Weise werden dabei die DNA-Segmente aus Mononukleotiden aufgebaut, wobei jedes beliebige geeignete Trägermaterial, vorzugsweise Glaspartikel und verschiedene Synthesestrategien, vorzugsweise das Triester- oder das Phosphit-triester-Verfahren, angewandt werden können.

Nachfolgend wird anhand der beiliegenden Zeichnungen ein Ausführungsbeispiel der Erfindung beschrieben. Es zeigen

Fig. 1 eine Vorrichtung mit zehn Reaktionsscheiben in perspektivischer Ansicht, teilweise aufgeschnitten,

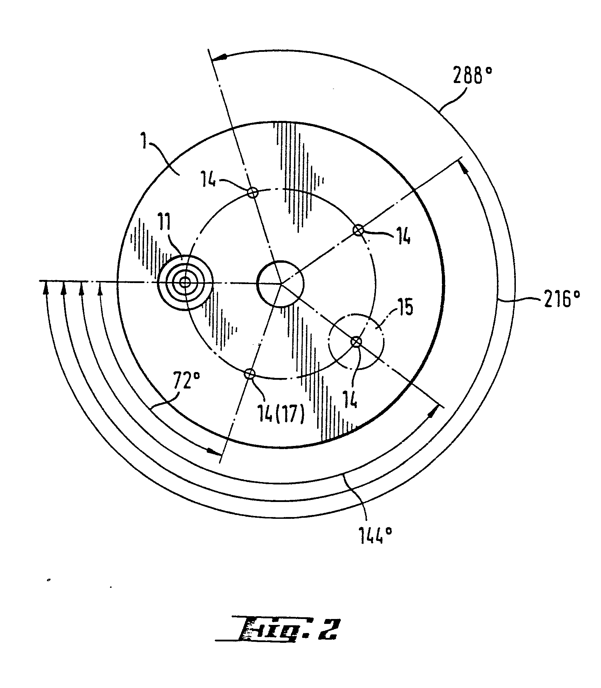

Fig. 2 eine Aufsicht auf eine einzelne Reaktionsscheibe.

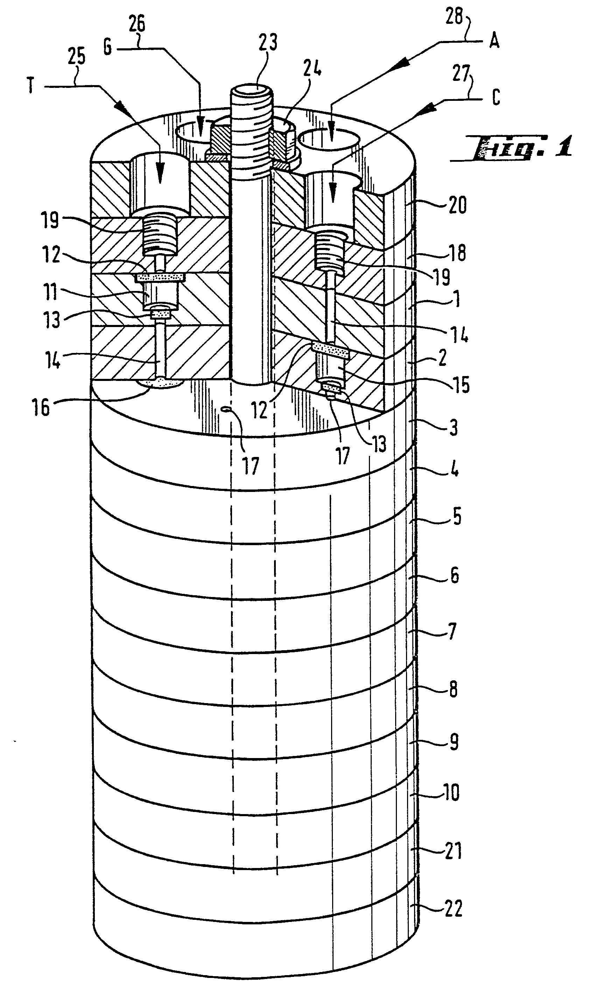

Fig. 1 zeigt eine Vorrichtung zur gleichzeitigen Synthese von zehn verschiedenen DNA-Segmenten, wobei als Träger ein Polymergranulat verwendet wird. Der jeweils erste Baustein der zu synthetisierenden Ketten befindet sich vor dem Synthesebeginn bereits auf dem Träger.

Die Vorrichtung besteht aus einem Stapel konzentrisch aufeinanderliegender runder Scheiben, in denen mehrere Kanalsysteme ausgebildet sind. Die sog. Reaktionsscheiben 1-10 sind den zehn zu synthetisierenden Oligonukleotiden zugeordnet, d. h. in jeder Reaktionsscheibe wird ein bestimmtes Oligonukleotid aufgebaut. Zu diesem Zweck weist beispielsweise die erste Reaktionsscheibe 1 eine Reaktionskammer 11 auf. Die Reaktionskammer 11 ist eine etwa halbwegs zwischen der Achse und dem Rand der Scheibe angeordnete, sich von der oberen Fläche bis zu etwa dreiviertel der Scheibendicke erstreckende Bohrung. Die Kammer dient zur Aufnahme des Trägergranulats. Am oberen Rand besitzt die Kammer eine umlaufende Verbreiterung zur Aufnahme einer Fritte 12, die die Reaktionskammer 11 nach oben abschliesst. In einer konzentrischen Ausnehmung des Kammerbodens befindet sich eine weitere Fritte 13, die den Abschluss der Kammer 11 nach unten gegenüber einer vom Kammerboden zur Unterfläche der Scheibe führenden, durchgehenden Bohrung mit sehr kleinem Durchmesser, z. B. 1 mm bildet.

Die Reaktionsscheibe 1 weist ausser der Reaktionskammer 11 vier durchgehende Bohrungen 14 von gleichem kleinem Durchmesser von z. B. 1 mm auf. Diese Bohrungen sind gegenüber der Reaktionskammer um jeweils 72°, 144°, 216° und 288° versetzt angeordnet.

Die weiteren Reaktionsscheiben sind in der gleichen Weise aufgebaut. Sie sind individuell gegeneinander um ein zentrales Verbindungselement drehbar.

Die unmittelbar anschliessende Reaktionsscheibe 2 ist in der Zeichnung um 144° gedreht gezeigt, so dass ihre Reaktionskammer 15 koaxial zur Bohrung 14 der ersten Scheibe 1 liegt.

Die nächste Reaktionsscheibe 3, die in der Zeichnung nicht mehr aufgeschnitten gezeigt ist, befindet sich wieder in der Lage, in der ihre Reaktionskammer -erkennbar an der Fritte 16- mit der Reaktionskammer 11 der Scheibe 1 fluchtet. Bei der Scheibe 3 ist eine um 72° versetzte Bohrung 17 erkennbar.

Den Stapel der Reaktionsscheiben 1-10 begrenzen eine obere Anschluss-Scheibe 18 zum Anschluss der Zuführungsschlauchleitungen für die Reagenzien und eine untere Anschluss-Scheibe 21 für den Anschluss der wegführenden Schlauchleitungen. Die obere Anschluss-Scheibe 18 weist mit den Kanälen bzw. Reaktionskammern in den Reaktionsscheiben fluchtende Bohrungen 19 mit Gewinden zum Einschrauben der Fittings für die Zuführungsschläuche. Die sich etwa bis zu dreiviertel der Scheibendicke erstreckenden Bohrungen 19 setzen sich, ähnlich wie bei den Reaktionskammern, in durchgehenden engen Bohrungen bis zur Plattenunterseite fort. Die untere Anschluss-Scheibe 21 kann der oberen entweder gleichen oder sie besitzt einen einfachen Sammelkanal (nicht gezeigt), falls die verbrauchten Reagenzien nicht mehr getrennt geführt werden müssen.

Die Reaktionsscheiben 1-10 und die Anschlussscheibe 18 weisen auf ihren unteren Flächen die engen Bohrungen ringförmig umgehende Nuten auf, die zur Aufnahme von O-Ringen dienen, mit denen die Kanäle an den Uebergängen zwischen den Scheiben abgedichtet werden. Diese Dichtungssysteme sind in der Zeichnung zugunsten der Uebersichtlichkeit weggelassen.

Ueber der oberen Anschluss-Scheibe 18 ist eine obere Anpress-Scheibe 20 angeordnet. Entsprechend befindet sich unter der unteren Anschluss-Scheibe 21 eine untere AnpressScheibe 22. Diese Anpress-Scheiben 20, 22 übertragen die Kraft die von einem als zentrales Verbindungselement dienenden Bolzen 23 erzeugt wird, der sich in einer durch den ganzen Stapel geführten axialen Bohrung befindet und mit einer Verschraubung (Mutter 24) versehen ist, auf den Stapel. Durch diese Presskraft werden die Scheiben derart aufeinandergedrückt, dass absolute Dichtheit der Kanäle gewährleistet ist und ein allfälliger, innerhalb der Dichtungsringe möglicher Totraum eliminiert wird.

Durch entsprechende Ausrichtung der Reaktionsscheiben 1-10 fluchten ihre Reaktionskammern und Bohrungen so, dass sich im Stapel vier Kanäle ergeben, von denen jetzt jeder mit einem der Mononukleotide A, T, C oder G beschickt wird (in der Zeichnung durch Pfeile angedeutet), wodurch die wachsenden DNA-Stücke simultan um das entsprechende Nukleotid verlängert werden.

Dazu wird die Reaktionskammer 11 von Scheibe 1 jeweils für die entsprechende Zeit in den Kanal gebracht, dessen Nukleotid angelagert werden soll (befindet sich in der Zeichnung im Kanal, in welchem mit T verlängert wird, während sich die Kammer von Scheibe 2 im C-Kanal befindet : hier würde also C in der gleichen Zeit wie bei Scheibe 1 T angelagert werden). Alle für die Anknüpfung der Mononukleotide erforderlichen Reaktionen und Waschprozesse (Schutzgruppenabspaltung, Capping, ggf. Oxidation) finden ebenfalls simultan statt (kontinuierliches Durchflussverfahren). Nach Beendigung eines Anknüpfungszyklus werden die einzelnen Scheiben wieder so verdreht, dass ihre Kammern sich im Kanal des nächsten jeweils anzulagernden Nukleotids befinden. Zum Verdrehen der Scheiben wird die Mutter 24 gelöst und anschliessend wieder angezogen. Ist die Synthese eines DNA-Fragmentes in einer der Scheiben beendet, so wird ihre Kammer in die Leerposition gestellt, wobei die restlichen vier engen Bohrungen der Scheibe die vier Verbindungskanäle für die Verlängerung der DNA-Segmente in den anderen Reaktionsscheiben aufrecht erhalten.

Nach Beendigung der Synthesen aller DNA-Segmente in den einzelnen Reaktionsscheiben wird aus ihnen nach Auseinanderbauen der Vorrichtung und Entfernen der Fritte 12 das Trägermaterial mit den anhaftenden synthetisierten Nukleotidsequenz entfernt. Nach Abspaltung der Schutzgruppen und Loslösen vom Trägermaterial werden die gewünschten ungeschützten DNA-Segmente aus den Rohgemischen mittels eines geeigneten Reinigungsmethode in reiner Form erhalten.

Um die individuelle Drehung der einzelnen Reaktionsscheiben auf die sinnvollen Winkelschritte von 72° zu beschränken, müssen mindestens sichtbare Markierungen angebracht werden. Besser ist allerdings ein Mechanismus der bei der richtigen Winkelstellung ein Einrasten vorsieht.

Die Vorrichtung kann sowohl manuell als auch mechanisch mit geeigneten Antriebsorganen bedient werden. Letztere Betriebsweise kann auch mit einer Programmsteuerung kombiniert werden.

Die vorstehend als Ausführungsbeispiel beschriebene Vorrichtung besteht aus Reaktionsscheiben mit ca. 60 mm Durchmesser und ca. 10 mm Dicke. Die Bohrungen 14 haben, wie bereits erwähnt, einen Durchmesser von ca. 1 mm. Die Reaktionskammern haben einen Durchmesser von ca. 6 mm. Diese Abmessungen haben aber lediglich im Zusammenhang mit einem bestimmten Syntheseprogramm Bedeutung. Vorrichtungen mit gleichem Aufbau- und Funktionsprinzip können in weiten Grenzen wählbare Abmessungen aufweisen und können insbesondere wesentlich grösser sein als das beschriebene Ausführungsbeispiel. Auch die Zahl der Bohrungen pro Scheibe kann grösser sein als bei dem vorstehend beschriebenen Beispiel. Dies ist beispielsweise für Peptidsynthesen wichtig.

Ausserdem ist selbstverständlich auch die Form der Scheiben und speziell der Reaktionskammern in keiner Weise auf das Ausführungsbeispiel beschränkt. So wäre es beispielsweise möglich, anstelle der kreisförmigen Anordnung der Bohrungen auf kreisrunden Scheiben eine lineare Anordnung der Bohrungen und dementsprechend eine lineare Verschiebung der Scheiben anstelle der Verdrehung vorzusehen.

**Patentansprüche** (für die Vertragsstaaten : BE, GB, NL, AT)

1. Vorrichtung zur Durchführung chemischer Reaktionssequenzen gekennzeichnet durch
   eine erste Anzahl n von Reaktionsscheiben (1), die stapelförmig übereinander angeordnet und individuell gegeneinander verschiebbar sind,
   Mittel zur Festlegung der Verschiebung in einer zweiten Anzahl m von gleichen Schritten,
   m in den Schrittabständen voneinander angeordnete durchgehende Bohrungen (11, 14) der Scheiben, von denen eine je Scheibe zu einer Reaktionskammer (14) erweitert ist,
   Mittel zum Zurückhalten eines Trägermaterials in der Reaktionskammer beim Durchfluss eines Reagenz.

2. Vorrichtung nach Anspruch 1, dadurch gekennzeichnet, dass die Reaktionsscheiben kreisförmig und gegeneinander verdrehbar und die Bohrungen ebenfalls kreisförmig auf ihnen angeordnet sind.

3. Vorrichtung nach Anspruch 2 dadurch gekennzeichnet, dass die Schrittabstände gleiche Winkelschritte sind.

4. Vorrichtung nach Anspruch 1, dadurch gekennzeichnet, dass die Mittel zum Zurückhalten des Trägermaterials in der Reaktionskammer Fritten sind, die in entsprechenden Ausnehmungen der Reaktionskammer angeordnet sind.

5. Vorrichtung nach einem der vorangehenden Ansprüche, dadurch gekennzeichnet, dass die Unterflächen der Reaktionsscheiben zu den Bohrungen konzentrisch umlaufende Nuten aufweisen, in denen O-Ringe angeordnet sind.

6. Vorrichtung nach einem der vorangehenden Ansprüche, dadurch gekennzeichnet, daß die Bohrungen (11, 14) linear angeordnet und die Reaktionsscheiben (1) linear gegeneinander verschiebbar sind.

**Patentansprüche** (für die Vertragsstaaten : DE, FR, IT, CH, SE)

1. Vorrichtung zur Durchführung chemischer Reaktionssequenzen mit

einer ersten Anzahl n von Reaktionsscheiben (1), die stapelförmig übereinander angeordnet und individuell gegeneinander verschiebbar sind,

Mitteln zur Festlegung der Verschiebung in einer zweiten Anzahl m von gleichen Schritten,

m in den Schrittabständen voneinander angeordneten durchgehenden Bohrungen (11, 14) der Scheiben, von denen eine je Scheibe zu einer Reaktionskammer (14) erweitert ist,

Mitteln zum Zurückhalten eines Trägermaterials in der Reaktionskammer beim Durchfluss eines Reagens, dadurch gekennzeichnet, dass die Unterflächen der Reaktionsscheiben zu den Bohrungen konzentrisch umlaufende Nuten aufweisen, in denen O-Ringe angeordnet sind.

2. Vorrichtung nach Anspruch 1, dadurch gekennzeichnet, dass die Bohrungen linear angeordnet und die Reaktionsscheiben linear gegeneinander verschiebbar sind.

**Claims** (for the Contracting States : BE, GB, NL, AT)

1. An apparatus for performing chemical reaction sequences, characterized by

a first number n of reaction discs (1) which are superposed in the form of a stack and are individually displaceable relative to one another,

means for defining the displacement in a second number m of identical steps,

m continuous bores (11, 14) disposed in the discs at the step distances from one another, one of the bores being widened out per disc to form a reaction chamber (14),

means for retaining a support material in the reaction chamber on the flow of a reagent therethrough.

2. An apparatus according to claim 1, characterized in that the reaction discs are circular and rotatable relative to one another and the bores are also disposed on them in the form of a circle.

3. An apparatus according to claim 2, characterized in that the step distances are identical angle steps.

4. An apparatus according to claim 1, characterized in that the means for retaining the support material in the reaction chamber are frits disposed in appropriate recesses in the reaction chamber.

5. An apparatus according to any one of the preceding claims, characterized in that the undersides of the reaction discs have grooves extending concentrically to the bores and containing O-rings.

6. An apparatus according to any one of the preceding claims. characterized in that the bores (11, 14) are linearly disposed and the reaction discs (1) are linearly displaceable relative to one another.

**Claims** (for the Contracting States : DE, FR, IT, CH, LI, SE)

1. An apparatus for performing chemical reaction sequences having

a first number n of reaction discs (1) which are superposed in the form of a stack and are individually displaceable relative to one another,

means for defining the displacement in a second number m of identical steps,

m continuous bores (11, 14) disposed in the discs at the step distances from one another, one of the bores being widened out per disc to form a reaction chamber (14),

means for retaining a support material in the reaction chamber on the flow of a reagent therethrough, characterized in that the undersides of the reaction discs have grooves extending concentrically to the bores and containing O-rings.

2. An apparatus according to claim 1, characterized in that the bores are linearly disposed and the reaction discs are linearly displaceable relative to one another.

**Revendications** (pour les Etats contractants : BE, GB, NL, AT)

1. Dispositif pour exécuter des séquences de réactions chimiques caractérisé par

un premier nombre n de plateaux de réaction (1), qui sont empilés les uns sur les autres et que l'on peut déplacer individuellement les uns par rapport aux autres,

des moyens pour déterminer le déplacement dans un deuxième nombre m de pas identiques,

m alésages des plateaux traversant ceux-ci, ces alésages (11, 14) étant séparés les uns des autres d'une distance de pas, un de ces alésages par plateau formant en s'élargissant une chambre de réaction (14),

des moyens pour retenir un support dans la chambre de réaction lors de la circulation d'un réactif.

2. Dispositif selon la revendication 1, caractérisé en ce que les plateaux de réaction ont une forme circulaire et peuvent se déplacer les uns par rapport aux autres et en ce que les alésages disposés dans ces plateaux ont également une forme circulaire.

3. Dispositif selon la revendication 2, caractérisé en ce que les distances de pas correspondent à des angles de pas égaux.

4. Dispositif selon la revendication 1, caractérisé en ce que les moyens pour retenir le support dans la chambre de réaction sont des produits frittés qui sont disposés dans des évidements correspondants de la chambre de réaction.

5. Dispositif selon l'une quelconque des revendications précédentes caractérisé en ce que les surfaces inférieures des plateaux de réaction présentent des rainures, concentriques aux alésages, autour de ceux-ci, dans lesquelles sont disposés des joints toriques.

6. Dispositif selon l'une quelconque des revendications précédentes caractérisé en ce que les alésages (11, 14) ont une disposition linéaire et en ce que les plateaux de réaction (1) peuvent être déplacés de façon linéaire les uns par rapport aux autres.

**Revendications** (pour les Etats contractants : DE, FR, IT, CH, LI, SE)

1. Dispositif pour exécuter des séquences de réactions chimiques avec

un premier nombre n de plateaux de réaction (1), qui sont empilés les uns sur les autres et que l'on peut déplacer individuellement les uns par rapport aux autres,

des moyens pour déterminer le déplacement dans un deuxième nombre m de pas identiques,

m alésages de plateaux traversant ceux-ci, ces alésages (11, 14) étant séparés les uns des autres d'une distance de pas, un de ces alésages par plateau formant en s'élargissant une chambre de réaction (14),

des moyens pour retenir un support dans la chambre de réaction lors de la circulation d'un réactif, caractérisé en ce que les surfaces inférieures des plateaux de réaction présentent des rainures, concentriques aux alésages, autour de ceux-ci, dans lesquelles sont disposés des joints toriques.

2. Dispositif selon la revendication 1, caractérisé en ce que les alésages ont une disposition linéaire et en ce que les plateaux de réaction peuvent être déplacés de façon linéaire les uns par rapport aux autres.

*Fig. 2*